# FASCICULE DE BREVET EUROPEEN

(11) **EP 1 498 103 B1**
(45) Date de publication et mention de la délivrance du brevet: **04.03.2009**
(21) Numéro de dépôt: 04291687.4
(22) Date de dépôt: 02.07.2004
(51) Int. Cl.: A61K 8/85, A61K 8/37, A61Q 1/02, A61Q 1/06

(54) **Composition cosmétique de soin ou de maquillage des matières kératiniques comprenant un polyester**
Kosmetische Zusammensetzung zur Pflege oder Schminke von keratinischen Materialen enthaltend ein Polyester
Keratinic materials care or make-up cosmetic composition containing a polyester

(30) Priorité: 17.07.2003 FR 0308730
(43) Date de publication de la demande: 19.01.2005
(73) Titulaire: L'ORÉAL, 75008 Paris (FR)
(72) Inventeur: Filippi, Vanina, 75015 Paris (FR)
(74) Mandataire: Boulard, Denis

(56) Documents cités:
- FR-A- 2 795 309
- FR-A- 2 829 924
- US-A1- 2003 077 234
- US-B1- 6 342 527
- US-B1- 6 670 441
- DATABASE CAPLUS [Online] XP002270863 extrait de STN Database accession no. 2002:490327
- DATABASE CAPLUS [Online] XP002270864 extrait de STN Database accession no. 2002:347324 & JP 2002 128629 A (KOSEI CO) 9 mai 2002 (2002-05-09)

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un polyester particulier. Cette composition possède des propriétés cosmétiques remarquables et confère en particulier au maquillage ou au soin des propriétés de non collant, de non migration, de brillance, de tenue et de confort.

La composition de l'invention peut en particulier constituer un produit de maquillage du corps, des lèvres ou des phanères d'êtres humains ayant en particulier des propriétés de soin. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Le demandeur a trouvé de façon surprenante que l'utilisation i) d'au moins un polyester résultant de l'estérification, par un acide polycarboxylique, d'un ester d'acide hydroxy carboxylique aliphatique, ledit ester comprenant au moins deux groupes hydroxyle et ledit acide polycarboxylique étant un dimère diacide formé à partir d'au moins un acide gras insaturé et ii) d'au moins un ester hydrocarboné différent dudit polyester, permet d'obtenir une composition confortable, non collante, non migrante, brillante et/ou de bonne tenue.

L'invention a donc pour objet une composition cosmétique, notamment de soin ou de maquillage des matières kératiniques, comprenant un milieu cosmétiquement acceptable contenant i) au moins un polyester résultant de l'estérification, par un acide polycarboxylique, d'un ester d'acide hydroxy carboxylique aliphatique, ledit ester comprenant au moins deux groupes hydroxyle, ledit acide polycarboxylique étant un dimère diacide formé à partir d'au moins un acide gras insaturé et ii) au moins un ester hydrocarboné différent dudit polyester avantageusement sous la forme d'une huile.

L'invention a également pour objet un procédé cosmétique pour conférer à un film de composition cosmétique des propriétés de tenue, de non collant, de non migration, de brillant et/ou de confort, consistant à introduire dans ladite composition i) au moins un polyester résultant de l'estérification, par un acide polycarboxylique, d'un ester d'acide hydroxy carboxylique aliphatique comprenant au moins deux groupes hydroxyle, ledit acide polycarboxylique étant un dimère diacide formé à partir d'au moins un acide gras insaturé et ii) au moins un ester hydrocarboné différent dudit polyester avantageusement sous la forme d'une huile.

L'invention a encore pour objet l'utilisation de l'association i) d'au moins un polyester résultant de l'estérification, par un acide polycarboxylique, d'un ester d'acide hydroxy carboxylique aliphatique comprenant au moins deux groupes hydroxyle, ledit aide polycarboxylique étant un dimère diacide formé à partir d'au moins un acide gras insaturé et ii) d'au moins un ester hydrocarboné différent dudit polyester avantageusement sous la forme d'une huile, dans une composition physiologiquement acceptable dotée de propriétés de tenue, de non collant, de non migration, de brillant et/ou de confort.

L'invention a enfin pour objet l'utilisation de l'association i) d'au moins un polyester résultant de l'estérification, par un acide polycarboxylique, d'un ester d'acide hydroxy carboxylique aliphatique comprenant au moins deux groupes hydroxyle, ledit acide polycarboxylique étant un dimère diacide formé à partir d'au moins un acide gras insaturé et ii) d'au moins un ester hydrocarboné différent dudit polyester avantageusement sous la forme d'une huile, dans une composition physiologiquement acceptable, comme agent pour conférer à ladite composition de propriétés de tenue, de non collant, de non migration, de brillant et/ou de confort.

### POLYESTER

Le polyester selon l'invention est un copolymère qui résulte de l'estérification, par un acide polycarboxylique d'un ester d'acide hydroxy carboxylique aliphatique (que l'on appellera par la suite «ester hydroxylé»).

Le polyester selon l'invention a de préférence un poids moléculaire compris entre 3000 et 7000 g/mol. Par exemple, le Risocast DA-L a une masse moléculaire en nombre comprise entre 3500 et 4000 g/mol et le Risocast DA-H a une masse moléculaire en nombre comprise entre 6000 et 6500 g/mol. Ces produits sont commercialisés par la société japonaise KOKYU ALCOHOL KOGYO.

Le ratio molaire entre l'acide polycarboxylique et l'ester hydroxylé utilisés pour préparer le polyester selon l'invention est de préférence compris entre 0,25 et 1. Par exemple, ce ratio est égal à 0,75 pour le Risocast DA-H, et ce ratio est égal à 0,5 pour le Risocast DAL.

On peut citer en particulier, comme polyester utilisable dans la composition selon l'invention :
- l'ester résultant de la réaction d'estérification de l'huile de ricin hydrogénée avec l'acide dilinoléïque dans les proportions 2 pour 1.

Le polyester de la présente invention est avantageusement un composé pâteux ou visqueux à température ambiante (25°C). Par "pâteux" au sens de la présente invention, on entend désigner un composé gras lipophile, à changement d'état solide/liquide réversible, et comportant à la température de 23°C une fraction liquide et une fraction solide.

Par composé pâteux au sens de l'invention, on entend un composé ayant de préférence une dureté à 20°C allant de 0,001 à 0,5 MPa, de préférence de 0,002 à 0,4 MPa.

A titre d'exemple, le Risocast DA-L a une dureté à 20°C de 0,04 MPa, une fraction liquide à 23°C égale à 82 %, et une fraction liquide à 32°C égale à 90 %.

Les quantités des différents ingrédients de la composition selon l'invention seront données en pourcentages en poids par rapport au poids total de ladite composition.

Le polyester de la composition selon la présente invention peut représenter de 1 à 99 % du poids total de la composition, de préférence de 1 à 75 %, et mieux de 5 à 60%.

Le polyester selon l'invention résulte de l'estérification
- d'un acide polycarboxylique, et
- d'un ester d'acide hydroxy carboxylique aliphatique (que l'on appellera par la suite «ester hydroxylé»).

### ESTER HYDROXYLE

L'ester d'acide hydroxy carboxylique aliphatique (ou ester hydroxylé) comprend au moins deux groupes hydroxyle.

L'ester hydroxylé est avantageusement issu de la réaction d'au moins un acide carboxylique aliphatique hydroxylé avec un polyol.

Ledit acide aliphatique hydroxylé comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptibles d'être estérifiés par la suite par l'acide polycarboxylique pour obtenir le polyester de la présente invention.

Ledit polyol peut comprendre de 2 à 40 atomes de carbone et mieux de 3 à 30 atomes de carbone. Le polyol est, de préférence, un polyol aliphatique. Avantageusement, le polyol n'est pas un ose.
Ledit polyol qui réagit avec l'acide hydroxylé décrit précédemment peut être partiellement ou totalement estérifié ; de façon avantageuse, le polyol est totalement estérifié.

De préférence, l'ester d'acide hydroxy carboxylique aliphatique est un ester d'acide gras hydroxylé tel que le reste acide gras comporte au moins 12 atomes de carbone, par exemple de 12 à 40 atomes de carbone, et mieux de 12 à 28 atomes de carbone.

L'ester d'acide hydroxy carboxylique aliphatique utilisable dans l'invention peut être choisi parmi :
a) les esters partiels ou totaux de monoacides carboxyliques aliphatiques mono hydroxylés linéaires saturés ;
b) les esters partiels ou totaux de monoacides carboxyliques aliphatiques mono hydroxylés insaturés tels que le triricinoléate de glycéryle (huile de ricin) ;
c) les esters partiels ou totaux de polyol aliphatique en C₂ à C₁₆ ayant réagi avec un mono ou un poly acide carboxylique aliphatique mono ou poly hydroxylé comme notamment les triglycérides, les esters du pentaérythritol, du triméthylol propane, du propylène glycol, du néopentyl glycol, du dipentaérythritol, du polyglycérol, les esters du sorbitol ;
et leurs mélanges.

Avantageusement lorsque l'ester d'acide hydroxy carboxylique aliphatique résulte de l'estérification d'un polyacide carboxylique aliphatique tels que ceux cités ci-dessus, il ne reste pas de groupement COOH résiduel non engagé dans une liaison ester.

L'ester d'acide hydroxy carboxylique aliphatique est de préférence choisi parmi les esters de polyols aliphatiques en C₂ à C₁₆, lesdits polyols ayant réagi avec un acide gras aliphatique hydroxylé à chaîne saturée ou insaturée, comportant au moins 12 atomes de carbone. L'acide gras est de préférence l'acide ricinoléique et l'ester d'acide hydroxy carboxylique aliphatique est de préférence l'huile de ricin hydrogénée.

### ACIDE POLYCARBOXYLIQUE

L'acide polycarboxylique comprend au moins deux groupes COOH. Il est un dimère diacide d'acide(s) carboxylique(s) aliphatique(s) insaturé(s).

Selon l'invention, l'acide polycarboxylique est un dimère diacide d'acide(s) gras insaturé(s), c'est-à-dire un dimère formé à partir d'au moins un acide gras insaturé, par exemple à partir d'un seul acide gras insaturé ou à partir de deux acides gras insaturés différents. L'acide gras est de préférence mono insaturé ou di insaturé. On entend par acide gras, un acide obtenu par hydrolyse de corps gras d'origine végétale ou animale.

Les dimères diacide d'acide(s) gras insaturé(s) ou encore dimère diacide sont classiquement obtenus par réaction de dimérisation intermoléculaire d'au moins un acide gras insaturé. De préférence, on dimérise un seul type d'acide gras insaturé.

Les dimères diacide d'acide(s) gras insaturé(s) sont notamment obtenus par la dimérisation d'un acide gras insaturé notamment en C₈ à C₃₄, notamment en C₁₂ à C₂₂, en particulier en C₁₆ à C₂₀, et plus particulièrement en C₁₈.

A titre représentatif de ces acides gras insaturés, on peut notamment citer l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

Le dimère diacide est de préférence saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles double liaisons en simples liaisons

Les dimères diacide d'acide(s) gras insaturé(s) préférés sont obtenus par dimérisation de l'acide linoléique, puis éventuellement par hydrogénation du dimère ainsi obtenu. La forme hydrogénée peut être partielle ou totale et notamment correspondre à la forme saturée plus stable à l'oxydation.

On trouve également dans le commerce des dimères diacides et notamment des diacides dilinoléiques dont la stabilité vis-à-vis de l'oxydation a été améliorée par hydrogénation des doubles liaisons restant après la réaction de dimérisation.

Dans la présente invention, on peut utiliser tout dimère diacide disponible actuellement dans le commerce.

### ESTER HYDROCARBONE

Par « ester hydrocarboné», on entend un composé différent du polyester décrit précédemment et comprenant au moins une fonction ester COO. Le mot ester selon l'invention signifie un monoester, un diester, un triester et plus généralement un polyester différent du polyester décrit précédemment.

L'ester hydrocarboné conforme à l'invention est de préférence une huile, c'est-à-dire un corps gras liquide à pression atmosphérique et à une température de 23°C.

L'ester hydrocarboné peut être linéaire, ramifié ou cyclique, saturé ou insaturé.

L'ester hydrocarboné est de préférence une huile non volatile.
Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10 -3 à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg)

En particulier, l'ester hydrocarboné peut répondre à la formule RCOOR' dans laquelle RCOO représente un reste d'acide carboxylique comportant de 2 à 30 atomes de carbone, et R' représente une chaîne hydrocarbonée contenant de 1 à 30 atomes de carbone.

RCOO est de préférence le reste d'un acide carboxylique aliphatique comprenant 2 à 30 atomes de carbone, de préférence de 4 à 26 atomes de carbone, de préférence encore de 4 à 22 atomes de carbone.

Le radical R est avantageusement un radical alkyle ou un radical alkényle. On entend par radical alkyle, un radical aliphatique constitué de carbone et d'hydrogène, linéaire ou ramifié, et saturé. On entend par radical alkényle un radical aliphatique constitué de carbone et d'hydrogène, linéaire ou ramifié, et insaturé, i.e. comportant au moins une double liaison carbone carbone, de préférence de une à trois doubles liaisons, de préférence une double liaison carbone carbone.

RCOO peut représenter le reste d'un acide gras, c'est-à-dire un acide obtenu par hydrolyse de corps gras d'origine végétale ou animale.

Le radical R'O représente le reste d'un alcool, de préférence un alcool aliphatique, saturé ou insaturé, linéaire ou ramifié. Le radical R' est avantageusement un radical alkyle ou un radical alkényle, indépendamment du choix du radical R, alkyle et alkényle étant définis comme précédemment.

R'O peut représenter le reste d'un alcool gras, c'est-à-dire d'un alcool obtenu par hydrogénation d'un acide gras tel que défini précédemment.

R'O est de préférence le reste d'un alcool aliphatique comprenant 2 à 30 atomes de carbone, de préférence de 4 à 26 atomes de carbone, de préférence encore de 4 à 22 atomes de carbone.

R e t R' sont choisis indépendamment l'un de l'autre. De préférence, on les choisit de manière à ce qu'ils soient tous les deux saturés et ramifiés, ou tous les deux linéaires et mono insaturés.

L'ester peut notamment comprendre jusqu'à 60 atomes de carbone, de préférence de 10 à 45, et de préférence de 18 à 40 atomes de carbone.

Selon un mode de mise en oeuvre, l'ester hydrocarboné est un mono ester ramifié et saturé. De préférence l'ester est un mono ester d'un acide carboxylique aliphatique ramifié et saturé et d'un alcool aliphatique ramifié et saturé.

Selon un autre mode de mise en oeuvre, l'ester hydrocarboné est un mono ester d'un acide gras mono linéaire et insaturé, et d'un alcool gras linéaire et mono insaturé (comportant une double liaison carbone carbone).

Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyl-2-docécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges..

L'isononanoate d'isononyle, l'érucate d'oléyle, le néopentanoate d'octyl-2-docécyle et leurs mélanges conviennent tout particulièrement à la réalisation de l'invention.

Cet ou ces ester(s) hydrocarboné(s) peut(peuvent) être utilisé(s) dans la composition à raison de 5 à 90 %, notamment de 10 à 60 % et en particulier de 20 à 50 % en poids par rapport au poids total de la composition.

### BRILLANCE DE LA COMPOSITION

D'une manière générale, les compositions cosmétiques et plus particulièrement celles destinées à être appliquées sur les lèvres peuvent être caractérisées par un indice de brillance moyenne.

Par « brillance moyenne », on entend la brillance telle qu'elle peut être mesurée à l'aide d'un brillancemètre, de manière conventionnelle, par la méthode suivante :
Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRI-GLOSS.

La brillance moyenne de la composition est avantageusement supérieure ou égale à 30, voire supérieure ou égale à 40, notamment supérieure ou égale à 50, en particulier supérieure ou égale à 60, plus particulièrement supérieure ou égale à 65 ou mieux supérieure ou égale à 70, notamment lorsque la composition est destinée à être appliquée sur les lèvres.

Par exemple, une composition cosmétique de type rouge à lèvres peut posséder une brillance moyenne égale à 60 environ, une composition cosmétique de type base de gloss liquide ou une composition cosmétique de type fard à paupières de brillance moyenne égale à 70 environ, et une composition cosmétique de type base de vernis à ongles de brillance moyenne égale à 50 environ.

### HUILE BRILLANTE

La composition selon l'invention contient avantageusement une huile de masse molaire élevée allant de 650 à 10000 g/mol différente du polyester lorsqu'il se présente sous la forme d'une huile, et de l'ester hydrocarboné décrits précédemment. Par « huile », on entend un composé non aqueux, non miscible à l'eau, liquide à température ambiante (25°C) et pression atmosphérique (760 mm de Hg).

L'huile utilisée dans la composition selon la présente invention a une masse molaire allant de 650 à 10000 g/mol, et de préférence entre 750 et 7500 g/mol.

En effet, les huiles de masse molaire trop faible, associées avec le polyester dans la composition selon l'invention, conduisent à des compositions qui ne sont pas assez brillantes ; les huiles ayant une masse molaire trop élevée donnent, elles, des compositions jugées trop collantes.

Ainsi, le triglycéride d'acides caprique/caprylique (tel que celui commercialisé ou fabriqué sous la référence ESTOL 3603 MCT OIL par la société Uniqema), qui a une masse molaire égale à 494 g/mol, conduit à des compositions ayant de moins bonnes propriétés cosmétiques que les huiles ayant une masse molaire allant de 650 à 10000 g/mol.

L'huile de masse molaire allant de 650 à 10000 g/mol utilisable dans la présente invention peut être choisie parmi :
- les polymères lipophiles tels que :
   - les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
   - les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
   - les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
   - les copolymères de la vinylpyrrolidone tels que : le copolymère vinylpyrrolidone/1-héxadécène, ANTARON V-216 commercialisé ou fabriqué par la société ISP (MM=7300 g/mol),
- les esters tels que :
   - les esters d'acides gras linéaires ayant un nombre total de carbone allant de 35 à 70 comme le tétrapélargonate de pentaérythrityle (MM=697,05 g/mol),
   - les esters hydroxylés tels que le triisostéarate de polyglycérol-2 (MM=965,58 g/mol),
   - les esters aromatiques tels que le tridécyl trimellitate (MM=757,19 g/mol),
   - les esters d'alcool gras ou d'acides gras ramifiés en C₂₄-C₂₈ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le t étraisostéarate d e p olyglycéryle -2 (MM=1232,04 g /mol) ou encore le tétra décyl -2 tétradécanoate de pentaérythrityle (MM=1538,66 g/mol),
- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

L'huile de masse molaire allant de 650 à 10000 g/mol utilisée dans la composition selon l'invention peut représenter de 1 à 99%, de préférence de 10 à 80%, et mieux de 5 à 70% du poids total de la composition.

### PATEUX

La composition peut également contenir un composé pâteux différent du polyester décrit précédemment.

Par "pâteux" au sens de la présente invention, on entend désigner un composé gras lipophile, à changement d'état solide/liquide réversible, et comportant à la température de 23°C une fraction liquide et une fraction solide.

Par composé pâteux au sens de l'invention, on entend un composé ayant de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi :
- la lanoline et ses dérivés,
- les composés fluorés polymères ou non,
- les composés siliconés polymères ou non,
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
   - les polyéthers liposolubles résultant de la polyéthérification entre un ou plusieurs diols en C₂-C₁₀₀, de préférence en C₂-C₅₀,
   - les esters,
et leurs mélanges.

Le composé pâteux est de préférence polymère, notamment hydrocarboné.

La composition est de préférence exempte de lanoline ou d'un de ses dérivés.

Un composé pâteux siliconé et fluoré préféré est le polyméthyl trifluoropropryl méthylalkyl diméthylsiloxane, fabriqué sous la dénomination X22-1088 par SHIN ETSU.

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les pâteux esters, on préfère notamment :
- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme
   - l'ester résultant de la réaction d'estérification de l'huile de ricin hydrogénée avec l'acide isostéarique dans les proportions 1 pour 1 (1/1) ou monoisostéarate d'huile de ricin hydrogénée,
   - l'ester résultant de la réaction d'estérification de l'huile de ricin hydrogénée avec l'acide isostéarique dans les proportions 1 pour 2 (1/2) ou le diisostéarate d'huile de ricin hydrogénée,
   - l'ester résultant de la réaction d'estérification de l'huile de ricin hydrogénée avec l'acide isostéarique dans les proportions 1 pour 3 (1/3) ou triisostéarate d'huile de ricin hydrogénée,
   - et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Le composé pâteux représente de préférence 1 à 99%, mieux 1 à 60%, mieux 2 à 30% et mieux encore 5 à 15% en poids de la composition.

### COLORANT

Avantageusement, la composition de l'invention peut comprendre, en outre, au moins une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.

Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire avantageusement colorée pouvant représenter de 0,001 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

La composition contient avantageusement des pigments goniochromatiques, par exemple des pigments multicouches interférentiels, et/ou des pigments réfléchissants. Ces deux types de pigments sont décrits dans la demande FR0209246 dont le contenu est incorporé par référence dans la présente demande.

### CHARGES

Les charges peuvent être présentes à raison de 0,001 à 35 % (si présentes) du poids total de la composition, de préférence 0,5 à 15 %. On peut notamment citer le talc, le mica, le kaolin, les poudres de Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl ® de Toshiba, par exemple), la sillice.

### CIRE

En particulier, elle contient, en outre, au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

La composition selon l'invention contient avantageusement de la cire de polyéthylène de masse moléculaire en poids comprise entre 300 et 700, notamment égale à 500 g/mol.

A titre indicatif, la cire peut représenter de 0,01 à 50 %, de préférence de 2 à 40 %, et mieux de 5 à 30 % du poids total de la composition.

### HUILES NON BRILLANTES

Les huiles additionnelles autres que les huiles de masse molaire allant de 650 à 10000 g/mol et autres que l'ester hydrocarboné selon l'invention peuvent être des huiles hydrocarbonées et/ou siliconées et/ou fluorées. Ces huiles peuvent être d'origine animale, végétale, minérale ou synthétique. "Par huile hydrocarbonée", on entend une huile comportant principalement des atomes de carbone et d'hydrogène et éventuellement une ou plusieurs fonctions choisies parmi les fonctions hydroxyle, ester, éther, carboxylique. A titre d'exemple d'huile additionnelle utilisable dans l'invention, on peut citer :
- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones (telles que la phényl triméthicone vendue sous le nom commercial DC556 par Dow Corning), les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- les acides gras ayant de 12 à 26 atomes de carbone comme l'acide oléïque ;
et leurs mélanges.

On utilise de préférence des huiles additionnelles d'origine végétale ou synthétique.

Les huiles additionnelles peuvent représenter de 0,001 à 90 % du poids total de la composition, de préférence de 0,05 à 60 % et mieux de 1 à 35 %.

### ADDITIFS

La composition de l'invention peut comprendre, en outre, tout additif complémentaire usuellement utilisé dans le domaine concerné, tel que de l'eau, des antioxydants, des conservateurs, des neutralisants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0,0005 à 20% du poids total de la composition et mieux de 0,001 à 10%.

Comme actif cosmétique utilisable dans l'invention, on peut citer les vitamines A, E, C, B₃, F, les provitamines comme le D-panthénol, la glycérine, les actifs apaisants comme l'α-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

### GALENIQUES

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 5 %.

La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains.
Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

### Exemple 1 et exemple 2 comparatif: Rouge à lèvres en stick

Dans l'exemple 2 comparatif, le RISOCAST DA-L a été substitué poids pour poids par la cire de lanoline oxypropylènée (commercialisée sous la référence EMERY 1695 par la société Cognis).

| **Phase** | **Matières premières** | **ESSAI 1 Essai d'invention** | **ESSAI 2 Essai comparatif** |
|---|---|---|---|
| **A-** | Erucate d'oleyle | 9 | 9 |
| | Isononanoate d'isononyle | 17,7 | 17,7 |
| | Esters d'acides gras végétaux isostéarique, adipique de glycéryle (Softisan®649) | 10,6 | 10,6 |
| | BHT | 0,06 | 0,06 |
| **B-** | Isononanoate d'isononyle | 4,16 | 4,16 |
| | Erucate d'oleyle | 4,16 | 4,16 |
| | Polylaurate de vinyle | 8 | 8 |
| | Cire de lanoline oxypropylénée | - | 9,01 |
| | Polyester d'acide dimère dilinoléïque et d'huile de ricin hydrogénée (RISOCAST DA-L®, société KOKYU ALCOHOL KOGYO) | 9,01 | - |
| | Esters d'acides gras végétaux isostéarique, adipique de glycéryle (Softisan® 649) | 3,44 | 3,44 |
| | Phosphate de tri-oleyle | 1 | 1 |
| | glycérine | 7,97 | 7,97 |
| | Hectorite modifiée par chlorure de di-stearyl dimethyl ammonium | 1,17 | 1,17 |
| | BHT | 0,06 | 0,06 |
| **C-** | Cire de polyethylène (MW 500) | 7 | 7 |
| | Cire de carnauba | 2,4 | 2,4 |
| **D-** | Silice pyrogénée hydrophobe | 1 | 1 |
| | Pigments | 8,7 | 8,7 |
| **E-** | Actifs | 4,06 | 4,06 |
| **F-** | Parfum | 0,5 | 0.5 |

### Mode opératoire :

- Broyer la phase D à la tricylindre dans la phase huileuse A.
- Préparer le gel d'hectorite modifiée, à l'aide d'un homogénéïsateur haute pression, avec tous les composés de la phase B sauf la glycérine.
- Ajouter le broyat de pigments, le gel de bentone et les cires (phase C) dans un poëlon. Chauffer à 100°C et homogénéïser à l'aide d'un raynerie (vitesse de rotation: 1500 RPM)
- Ajouter la glycérine et augmenter la vitesse de rotation à 3200 RPM. Agiter pendant 10 minutes puis diminuer la vitesse de rotation à 1500 RPM.
- Ajouter les actifs et le parfum 5 minutes avant le coulage.
- Couler dans un moule à 42°C que l'on place au congélateur à -20°C pendant une demi heure avant de procéder au démoulage des sticks.

### Evaluation cosmétique :

Les formules ont été évaluées en salle de maquillage par 12 femmes suivant différents critères sensoriels.
L'exemple 1 est plus brillant à l'application et à une heure, développe davantage la couleur, et donne une sensation de gras moindre que l'exemple 2 comparatif.

Une évaluation instrumentale a également été réalisée sur 6 femmes.

| **PARAMETRES INSTRUMENTAUX** | | |
|---|---|---|
| **FORMULES** | **Exemple 2 comparatif** | **Exemple 1** |
| **tenue de la couleur à 1 heure (%)** | 88,0 | 90,0 |
| **tenue de la couleur après épreuves (%)** | 44,0 | 53,0 |

L'exemple 1 a une meilleure tenue de la couleur après épreuves que l'exemple 2 comparatif contenant la cire de lanoline oxypropylènée

### Exemple 3 et exemple 4 comparatif: Rouge à lèvres en stick

Dans l'exemple 4 comparatif, le RISOCAST DA-L a été substitué par la lanoline acétylée (commercialisée sous la référence ACETADEPS P80 par la société Croda).

| **Phase** | **Matières premières** | **Exemple 3** | **Exemple 4 comparatif** |
|---|---|---|---|
| **A-** | Esters d'acides gras végétaux iso-stéarique adipique de glycéryle (Softisan®649) | 14,98 | 14,98 |
| | Polyester d'acide dimère dilinoléïque et d'huile de ricin hydrogénée (Risocast DA-L®, société KOKYU ALCOHOL KOGYO) | 10,21 | - |
| | Lanoline acétylée | - | 10,21 |
| | Néopentanoate d'octyl-2 dodecyle | 19,06 | 19,06 |
| | Isoparaffine hydrogénée | 6,6 | 6,6 |
| | Triglycérides d'acides caprylique / caprique | 10,21 | 21,21 |
| | Phenyl trimethyl siloxy trisiloxane (visco 20 cst) | 6,94 | 6,94 |
| | BHT | 0,07 | 0,07 |
| | Polybutylène | 15,25 | 15,25 |
| **B-** | Cire de polyéthylène (MW 500) | 7,52 | 7,52 |
| | Cire microcristalline | 2,6 | 2,6 |
| **C-** | Pigments | 4,44 | 4,44 |
| **D-** | Copolymère de di-methacrylate d'ethyle glycol / methacrylate de lauryle | 0,92 | 0,92 |
| **E-** | Phase active | 1,2 | 1,2 |

### Mode opératoire :

- Broyer la phase C dans la phase huileuse A.
- Ajouter le broyat, les cires (phase B) et la phase D dans un poëlon. Chauffer à 100°C et homogénéïser à l'aide d'un raynerie.
- 5 minutes avant le coulage ajouter la phase active E.
- Couler dans un moule à 42°C, placer dans un réfrigérateur à -20°C pendant une demi heure avant de procéder au démoulage des sticks.

### Evaluation cosmétique :

Les formules ont été évaluées en salle de maquillage par 12 femmes suivant différents critères:

Le stick de l'exemple 3 est moins collant à l'application et au bout d'une heure et migre moins.

## Revendications

1. Composition cosmétique comprenant un milieu cosmétiquement acceptable contenant i) au moins un polyester résultant de l'estérification, par un acide polycarboxylique, d'un ester d'acide hydroxy carboxylique aliphatique, ledit ester comprenant au moins deux groupes hydroxyle, ledit acide polycarboxylique étant un dimère diacide formé à partir d'au moins un acide gras insaturé et ii) au moins un ester hydrocarboné différent dudit polyester.

2. Composition selon la revendication précédente **caractérisée en ce que** le dimère est un dimère d'un acide gras insaturé en C₈ à C₃₄, notamment en C₁₂ à C₂₂ en particulier en C₁₆ à C₂₀ et plus particulièrement en C₁₈.

3. Composition selon la revendication précédente, **caractérisée en ce que** le ou les acides gras insaturés sont choisis parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

4. Composition selon la revendication 3, **caractérisée en ce que** l'acide gras insaturé est l'acide linoléique, et que le dimère est l'acide dilinoléïque.

5. Composition selon l'une quelconque des revendications précédentes, **caractérisée en ce que** le dimère diacide est saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles double liaisons en simples liaisons.

6. Composition selon l'une des revendications précédentes, **caractérisée en ce que** l'ester d'acide hydroxy carboxylique aliphatique est choisi parmi :
a) les esters partiels ou totaux de monoacides carboxyliques aliphatiques mono hydroxylés linéaires saturés ;
b) les esters partiels ou totaux de monoacides carboxyliques aliphatiques mono hydroxylés insaturés ;
c) les esters partiels ou totaux de polyols aliphatiques en C₂ à C₁₆ ayant réagi avec un mono ou un poly acide carboxylique aliphatique mono ou poly hydroxylé ;
et leurs mélanges.

7. Composition selon l'une des revendications précédentes, **caractérisée en ce que** l'ester d'acide hydroxy carboxylique aliphatique est choisi parmi les esters de polyols aliphatiques en C₂ à C₁₆, lesdits polyols ayant réagi avec un acide gras aliphatique hydroxylé à chaîne saturée ou insaturée, comportant au moins 12 atomes de carbone.

8. Composition selon l'une des revendications précédentes, **caractérisée en ce que** l'ester d'acide hydroxy carboxylique aliphatique est choisi parmi les esters saturés ou insaturés, de polyols aliphatiques en C₂ à C₁₆, lesdits polyols ayant réagi avec l'acide ricinoléique.

9. Composition selon l'une des revendications précédentes, **caractérisée en ce que** l'ester d'acide hydroxy carboxylique aliphatique est l'huile de ricin hydrogénée.

10. Composition selon l'une des revendications précédentes, **caractérisée en ce que** le polyester présente une fraction liquide et une fraction solide à 23°C et une dureté allant de 0,001 à 0,5 MPa, de préférence de 0,002 à 0,4 MPa.

11. Composition selon l'une des revendications précédentes, **caractérisée en ce que** le polyester représente de 1 à 99%, de préférence de 1 à 75 %, et mieux de 5 à 60% du poids total de la composition.

12. Composition selon l'une des revendications précédentes, **caractérisée en ce que** l'ester hydrocarboné est une huile.

13. Composition selon l'une des revendications précédentes, **caractérisée en ce que** l'ester hydrocarboné est un mono ester ramifié et saturé.

14. Composition selon l'une des revendications 1 à 12, **caractérisée en ce que** l'ester hydrocarboné est un mono ester d'un acide gras linéaire mono insaturé et d'un alcool gras linéaire et mono insaturé.

15. Composition selon l'une des revendications précédentes, **caractérisée en ce que** l'ester hydrocarboné comprendre de 10 à 60, de préférence de 10 à 45, de préférence de 18 à 40 atomes de carbone.

16. Composition selon l'une des revendications précédentes, **caractérisée en ce que** l'ester est choisi les esters de l'acide néopentanoïque comme le néopentanoate d'isodécyle, le néopentanoate d'isotridecyle, le néopentanoate d'isostéaryle, le néopentanoate d'octyl-2-docécyle, les esters de l'acide isononanoïque comme l'isononanoate d'isononyle, l'isononanoate d'octyle, l'isononanoate d'isodecyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges..

17. Composition selon l'une des revendications précédentes, **caractérisée en ce que** l'ester hydrocarboné est choisi parmi l'isononanoate d'isononyle, l'érucate d'oléyle, le néopentanoate d'octyl-2-docécyle et leurs mélanges.

18. Composition selon l'une des revendications précédentes, **caractérisée en ce qu'**elle se présente sous forme d'un rouge à lèvres ou d'un brillant à lèvres, d'un fard à joues ou à paupières, d'un mascara, d'un eye-liner, d'un vernis à ongles, d'un produit de bronzage artificiel de la peau, d'un produit de coloration ou de soin des cheveux.

19. Composition selon l'une des revendications précédentes, **caractérisée en ce qu'**elle comprend, en outre, au moins une matière colorante.

20. Composition selon l'une des revendications précédentes, **caractérisée en ce qu'**elle contient au moins une cire de polyéthylène.

21. Composition selon la revendication précédente, **caractérisée en ce que** la brillance moyenne de la composition est supérieure ou égale à 30, voire supérieure ou égale à 40, notamment supérieure ou égale à 50, en particulier supérieure ou égale à 60, plus particulièrement supérieure ou égale à 65 ou mieux supérieure ou égale à 70.

22. Composition selon l'une des revendications précédentes, **caractérisée en ce qu'**elle comprend, en outre, au moins un additif complémentaire choisi parmi l'eau, les antioxydants, les conservateurs, les neutralisants, les gélifiants lipophiles ou de composés non aqueux liquides, les gélifiants de phase aqueuse, les dispersants, les actifs cosmétiques et leurs mélanges.

23. Composition selon l'une des revendications précédentes, **caractérisée en ce qu'**elle se présente sous forme coulée ou compactée.

24. Composition selon l'une des revendications précédentes, **caractérisée en ce qu'**elle se présente sous forme d'une phase continue huileuse.

25. Composition selon l'une des revendications précédentes, **caractérisée en ce qu'**elle se présente sous forme anhydre.

26. Composition selon l'une des revendications précédentes, **caractérisée en ce qu'**elle se présente sous forme d'un rouge à lèvres ou d'un brillant à lèvres.

27. Procédé cosmétique pour conférer à un film de composition cosmétique des propriétés de non collant, de non migration, de brillant et/ou de confort, consistant à introduire dans ladite composition i) au moins un polyester de l'estérification, par un acide polycarboxylique, d'un ester d'acide hydroxylé carboxylique aliphatique comprenant au moins deux groupes hydroxyle, ledit acide polycarboxylique étant un dimère diacide formé à partir d'au moins un acide gras insaturé, et ii) au moins un ester hydrocarboné différent dudit polyester.

28. Utilisation de l'association i) d'au moins un polyester résultant de l'estérification, par un acide polycarboxylique, d'un ester d'acide hydroxy carboxylique aliphatique comprenant au moins deux groupes hydroxyle, ledit acide polycarboxylique étant un dimère diacide formé à partir d'au moins un acide gras insaturé, et ii) d'au moins un ester hydrocarboné différent dudit polyester dans une composition physiologiquement acceptable, non collante, non migrante, brillante, de bonne tenue et/ou confortable.

29. Utilisation de l'association i) d'au moins un polyester résultant de l'estérification, par un acide polycarboxylique aliphatique, d'un ester d'acide hydroxy carboxylique comprenant au moins deux groupes hydroxyle aliphatique, ledit acide polycarboxylique étant un dimère diacide formé à partir d'au moins un acide gras insaturé, et ii) d'au moins un ester hydrocarboné différent dudit polyester, dans une composition physiologiquement acceptable, comme agent pour conférer à ladite composition des propriétés de tenue, de non collant, de non migration, de brillant et/ou de confort.

## Claims

1. Cosmetic composition, comprising a cosmetically acceptable medium containing i) at least one polyester resulting from the esterification, with a polycarboxylic acid, of an aliphatic hydroxycarboxylic acid ester, the said ester comprising at least two hydroxyl groups, the said polycarboxylic acid being a diacid dimer formed from at least one unsaturated fatty acid, and ii) at least one hydrocarbon-based ester other than the said polyester.

2. Composition according to the preceding claim, **characterized in that** the dimer is an unsaturated C₈ to C₃₄, especially C₁₂ to C₂₂, in particular C₁₆ to C₂₀ and more particularly C₁₈ fatty acid dimer.

3. Composition according to the preceding claim, **characterized in that** the unsaturated fatty acid(s) is(are) chosen from undecenoic acid, linderic acid, myristoleic acid, palmitoleic acid, oleic acid, linoleic acid, elaidinic acid, gadolenoic acid, eicosapentaenoic acid, docosahexaenoic acid, erucic acid, brassidic acid and arachidonic acid, and mixtures thereof.

4. Composition according to Claim 3, **characterized in that** the unsaturated fatty acid is linoleic acid and the dimer is dilinoleic acid.

5. Composition according to any one of the preceding claims, **characterized in that** the diacid dimer is saturated, i.e. it contains no carbon-carbon double bonds, and it is obtained by condensation of unsaturated fatty acid(s) optionally followed by a hydrogenation, to convert any double bonds into single bonds.

6. Composition according to one of the preceding claims, **characterized in that** the aliphatic hydroxycarboxylic acid ester is chosen from:
a) partial or total esters of saturated linear monohydroxylated aliphatic monocarboxylic acids;
b) partial or total esters of unsaturated monohydroxylated aliphatic monocarboxylic acids;
c) partial or total esters of a C₂ to C₁₆ aliphatic polyol that has reacted with a monohydroxylated or polyhydroxylated aliphatic monocarboxylic or polycarboxylic acid;
and mixtures thereof.

7. Composition according to one of the preceding claims, **characterized in that** the aliphatic hydroxycarboxylic acid ester is chosen from esters of C₂ to C₁₆ aliphatic polyols, the said polyols having reacted with a hydroxylated aliphatic fatty acid with a saturated or unsaturated chain, containing at least 12 carbon atoms.

8. Composition according to one of the preceding claims, **characterized in that** the aliphatic hydroxycarboxylic acid ester is chosen from saturated or unsaturated esters of C₂ to C₁₆ aliphatic polyols, the said polyols having reacted with ricinoleic acid.

9. Composition according to one of the preceding claims, **characterized in that** the aliphatic hydroxycarboxylic acid ester is hydrogenated castor oil.

10. Composition according to one of the preceding claims, **characterized in that** the polyester has a liquid fraction and a solid fraction at 23°C and a hardness ranging from 0.001 to 0.5 MPa and preferably from 0.002 to 0.4 MPa.

11. Composition according to one of the preceding claims, **characterized in that** the polyester represents from 1% to 99%, preferably from 1% to 75% and better still from 5% to 60% of the total weight of the composition.

12. Composition according to one of the preceding claims, **characterized in that** the hydrocarbon-based ester is an oil.

13. Composition according to one of the preceding claims, **characterized in that** the hydrocarbon-based ester is a branched and saturated monoester.

14. Composition according to one of Claims 1 to 12, **characterized in that** the hydrocarbon-based ester is a monoester of a linear monounsaturated fatty acid and of a linear monounsaturated fatty alcohol.

15. Composition according to one of the preceding claims, **characterized in that** the hydrocarbon-based ester contains from 10 to 60, preferably from 10 to 45 and preferentially from 18 to 40 carbon atoms.

16. Composition according to one of the preceding claims, **characterized in that** the ester is chosen from neopentanoic acid esters, for instance isodecyl neopentanoate, isotridecyl neopentanoate, isostearyl neopentanoate and 2-octyldodecyl neopentanoate, isononanoic acid esters, for instance isononyl isononanoate, octyl isononanoate, isodecyl isononanoate, isotridecyl isononanoate and isostearyl isononanoate, but also isopropyl alcohol esters, such as isopropyl myristate, isopropyl palmitate, isopropyl stearate or isostearate, cetyl octanoate, tridecyl octanoate, 2-ethylhexyl 4-diheptanoate and palmitate, alkyl benzoate, polyethylene glycol diheptanoate and propylene glycol diethyl 2-hexanoate, and mixtures thereof. The said ester may also be chosen from synthetic esters, especially of a fatty acid, for instance purcellin oil, isopropyl myristate, ethyl palmitate or octyl stearate; hydroxylated esters, for instance isostearyl lactate, octyl hydroxystearate, diisopropyl adipate, and fatty alcohol heptanoates, octanoates and decanoates, and mixtures thereof.

17. Composition according to one of the preceding claims, **characterized in that** the hydrocarbon-based ester is chosen from isononyl isononanoate, oleyl erucate and 2-octyldodecyl neopentanoate, and mixtures thereof.

18. Composition according to one of the preceding claims, **characterized in that** it is in the form of a lipstick, a lip gloss, a makeup rouge, an eyeshadow, a mascara, an eyeliner, a nail varnish, an artificial tanning product for the skin, a hair colouring product or a haircare product.

19. Composition according to one of the preceding claims, **characterized in that** it also comprises at least one dyestuff.

20. Composition according to one of the preceding claims, **characterized in that** it contains at least one polyethylene wax.

21. Composition according to the preceding claim, **characterized in that** the mean gloss of the composition is greater than or equal to 30, or even greater than or equal to 40, especially greater than or equal to 50, in particular greater than or equal to 60, more particularly greater than or equal to 65, or better still greater than or equal to 70.

22. Composition according to one of the preceding claims, **characterized in that** it also comprises at least one additional additive chosen from water, antioxidants, preserving agents, neutralizers, lipophilic gelling agents or non-aqueous liquid compounds, aqueous phase-gelling agents, dispersants and cosmetic active agents, and mixtures thereof.

23. Composition according to one of the preceding claims, **characterized in that** it is in cast or compacted form.

24. Composition according to one of the preceding claims, **characterized in that** it is in the form of an oily continuous phase.

25. Composition according to one of the preceding claims, **characterized in that** it is in anhydrous form.

26. Composition according to one of the preceding claims, **characterized in that** it is in the form of a lipstick or a lip gloss.

27. Cosmetic process for giving a film of cosmetic composition tack-resistance, migration-resistance, gloss and/or comfort properties, which consists in introducing into the said composition i) at least one polyester resulting from the esterification, with a polycarboxylic acid, of an aliphatic hydroxycarboxylic acid ester comprising at least two hydroxyl groups, the said polycarboxylic acid being a diacid dimer formed from at least one unsaturated fatty acid, and ii) at least one hydrocarbon-based ester other than the said polyester.

28. Use of the combination i) of at least one polyester resulting from the esterification, with a polycarboxylic acid, of an aliphatic hydroxycarboxylic acid ester comprising at least two hydroxyl groups, the said polycarboxylic acid being a diacid dimer formed from at least one unsaturated fatty acid, and ii) of at least one hydrocarbon-based ester other than the said polyester, in a physiologically acceptable composition which has tack-resistance, migration-resistance, gloss, good staying-power and/or comfort properties.

29. Use of the combination i) of at least one polyester resulting from the esterification, with an aliphatic polycarboxylic acid, of an aliphatic hydroxycarboxylic acid ester comprising at least two hydroxyl groups, the said polycarboxylic acid being a diacid dimer formed from at least one unsaturated fatty acid, and ii) of at least one hydrocarbon-based ester other than the said polyester, in a physiologically acceptable composition, as an agent for giving the said composition staying-power, tack-resistance, migration-resistance, gloss and/or comfort properties.

## Patentansprüche

1. Kosmetische Zusammensetzung, die in einem kosmetisch akzeptablen Medium enthält: i) mindestens einen Polyester, der durch Veresterung eines aliphatischen Hydroxycarbonsäureesters mit einer Polycarbonsäure gebildet wird, wobei der Ester mindestens zwei Hydroxygruppen enthält und wobei die Polycarbonsäure ein Disäuredimer ist, das ausgehend von mindestens einer ungesättigten Fettsäure gebildet wird, und ii) mindestens einen Kohlenwasserstoffester, der von dem Polyester verschieden ist.

2. Zusammensetzung nach dem vorhergehenden Anspruch, **dadurch gekennzeichnet, dass** das Dimer ein Dimer einer ungesättigten C₈₋₃₄-Fettsäure und insbesondere einer Fettsäure mit 12 bis 22 Kohlenstoffatomen, besonders 16 bis 20 Kohlenstoffatomen und insbesondere 18 Kohlenstoffatomen ist.

3. Zusammensetzung nach dem vorhergehenden Anspruch, **dadurch gekennzeichnet, dass** die ungesättigte Fettsäure oder die ungesättigten Fettsäuren ausgewählt sind unter Undecensäure, Linderinsäure, Myristoleinsäure, Palmitoleinsäure, Ölsäure, Linolsäure, Elaidinsäure, Gadolensäure, Eicosapentaensäure, Docosahexaensäure, Erucasäure, Brassidinsäure, Arachidonsäure und deren Gemischen ausgewählt ist.

4. Zusammensetzung nach Anspruch 3, **dadurch gekennzeichnet, dass** es sich bei der ungesättigten Fettsäure um Linolsäure handelt und **dadurch**, dass das Dimer die Dilinolsäure ist.

5. Zusammensetzung nach einem der vorhergehenden Ansprüche, **dadurch gekennzeichnet, dass** das Disäuredimer gesättigt ist, d. h., dass es keine Kohlenstoff-Kohlenstoff-Doppelbindung aufweist und durch Kondensation einer oder mehrerer ungesättigter Fettsäuren und einer gegebenenfalls anschließend durchgeführten Hydrierung gebildet wird, um die gegebenenfalls enthaltenen Doppelbindungen in Einfachbindungen umzuwandeln.

6. Zusammensetzung nach einem der vorhergehenden Ansprüche, **dadurch gekennzeichnet, dass** der aliphatische Hydroxycarbonsäureester ausgewählt ist unter:
a) partiellen oder vollständigen Estern von monohydroxylierten, linearen, gesättigten, aliphatischen Monocarbonsäuren;
b) partiellen oder vollständigen Estern von monohydroxylierten ungesättigten aliphatischen Monocarbonsäuren;
c) partiellen oder vollständigen Estern von aliphatischen C₂₋₁₆-Polyolen, die mit einer aliphatischen ein- oder mehrfach hydroxylierten Mono- oder Polycarbonsäure umgesetzt wurden;
und deren Gemischen.

7. Zusammensetzung nach einem der vorhergehenden Ansprüche, **dadurch gekennzeichnet, dass** der Ester einer aliphatischen Hydroxycarbonsäure unter den Estern von aliphatischen C₂₋₁₆-Polyolen ausgewählt ist, wobei die Polyole mit einer hydroxylierten aliphatischen Fettsäure mit gesättigter oder ungesättigter Kette umgesetzt wurden, die mindestens 12 Kohlenstoffatome aufweist.

8. Zusammensetzung nach einem der vorhergehenden Ansprüche, **dadurch gekennzeichnet, dass** der aliphatische Hydroxycarbonsäureester unter den gesättigten oder ungesättigten Estern von aliphatischen C₂₋₁₆-Polyolen ausgewählt ist, wobei die Polyole mit Ricinolsäure umgesetzt wurden.

9. Zusammensetzung nach einem der vorhergehenden Ansprüche, **dadurch gekennzeichnet, dass** es sich bei dem aliphatischen Hydroxycarbonsäureester um hydriertes Ricinusöl handelt.

10. Zusammensetzung nach einem der vorhergehenden Ansprüche, **dadurch gekennzeichnet, dass** der Polyester eine flüssige Fraktion und eine bei 23 °C feste Fraktion aufweist und eine Härte besitzt, die im Bereich von 0,001 bis 0,5 MPa und vorzugsweise 0,002 bis 0,4 MPa liegt.

11. Zusammensetzung nach einem der vorhergehenden Ansprüche, **dadurch gekennzeichnet, dass** der Polyester 1 bis 99 %, vorzugsweise 1 bis 75 % und besser 5 bis 60 % des Gesamtgewichts der Zusammensetzung ausmacht.

12. Zusammensetzung nach einem der vorhergehenden Ansprüche, **dadurch gekennzeichnet, dass** es sich bei dem Kohlenwasserstoffester um ein Öl handelt.

13. Zusammensetzung nach einem der vorhergehenden Ansprüche, **dadurch gekennzeichnet, dass** der Kohlenwasserstoffester ein verzweigter und gesättigter Monoester ist.

14. Zusammensetzung nach einem der Ansprüche 1 bis 12, **dadurch gekennzeichnet, dass** es sich bei dem Kohlenwasserstoffester um einen Monoester einer linearen einfach ungesättigten Fettsäure und eines linearen und einfach ungesättigten Fettalkohols handelt.

15. Zusammensetzung nach einem der vorhergehenden Ansprüche, **dadurch gekennzeichnet, dass** der Kohlenwasserstoffester 10 bis 60 Kohlenstoffatome, vorzugsweise 10 bis 45 Kohlenstoffatome und noch bevorzugter 18 bis 40 Kohlenstoffatome aufweist.

16. Zusammensetzung nach einem der vorhergehenden Ansprüche, **dadurch gekennzeichnet, dass** der Ester unter den Estern von Neopentansäure, wie Isodecylneopentanoat, Isotridecylneopentanoat, Isostearylneopentanoat, 2-Octyldodecylneopentanoat, Estern von Isononansäure, wie Isononylisononanoat, Octylisononanoat, Isodecylisononanoat, Isotridecylisononanoat, Isostearylisononanoat, jedoch auch unter den Estern von Isopropylalkohol, wie Isopropylmyristat, Isopropylpalmitat, Isopropylstearat, Isopropylisostearat, Cetyloctanoat, Tridecyloctanoat, 2-Ethylhexyl-4-diheptanoat, 2-Ethylhexylpalmitat, Alkylbenzoat, Polyethylenglycoldiheptanoat, Propylenglycol-2-diethylhexanoat und deren Gemischen ausgewählt ist; wobei der Ester auch unter den synthetischen Estern ausgewählt sein kann, insbesondere einer Fettsäure, wie Purcellinöl, Isopropylmyristat, Ethylpalmitat, Octylstearat; hydroxylierten Estern, wie Isostearyllactat, Octylhydroxystearat, Diisopropyladipat, Heptanoaten, Octanoaten und Decanoaten von Fettalkoholen und deren Gemischen ausgewählt ist.

17. Zusammensetzung nach einem der vorhergehenden Ansprüche, **dadurch gekennzeichnet, dass** der Kohlenrwasserstoffester unter Isononylisononanoat, Oleylerucat, 2-Octyldodecylneopentanoat und deren Gemischen ausgewählt ist.

18. Zusammensetzung nach einem der vorhergehenden Ansprüche, **dadurch gekennzeichnet, dass** sie in Form eines Lippenstiftes oder als Lipgloss, als Wangenrouge oder Lidschatten, Mascara, Eyeliner, Nagellack, Produkt für die künstliche Braunfärbung der Haut, Produkt für die Färbung oder Pflege der Haare vorliegt,

19. Zusammensetzung nach einem der vorhergehenden Ansprüche, **dadurch gekennzeichnet, dass** sie ferner ein Farbmittel enthält.

20. Zusammensetzung nach einem der vorhergehenden Ansprüche, **dadurch gekennzeichnet, dass** sie mindestens ein Polyethylenwachs enthält.

21. Zusammensetzung nach dem vorhergehenden Anspruch, **dadurch gekennzeichnet, dass** der mittlere Glanz der Zusammensetzung größer oder gleich 30, sogar größer oder gleich 40, insbesondere größer oder gleich 50, besonders größer oder gleich 60, ganz besonders größer oder gleich 65 oder besser größer oder gleich 70 ist.

22. Zusammensetzung nach einem der vorhergehenden Ansprüche, **dadurch gekennzeichnet, dass** sie ferner mindestens ein zusätzliches Additiv enthält, das unter Wasser, Antioxidantien, Konservierungsmitteln, Neutralisationsmitteln, lipophilen Gelbildnern oder nichtwässrigen flüssigen Verwindungen, Gelbildnern für die wässrige Phase, Dispergiermitteln, kosmetischen Wirkstoffen und deren Gemischen ausgewählt ist.

23. Zusammensetzung nach einem der vorhergehenden Ansprüche, **dadurch gekennzeichnet, dass** sie in gegossener oder kompaktierter Form vorliegt.

24. Zusammensetzung nach einem der vorhergehenden Ansprüche, **dadurch gekennzeichnet, dass** sie in Form einer kontinuierlichen Ölphase vorliegt.

25. Zusammensetzung nach einem der vorhergehenden Ansprüche, **dadurch gekennzeichnet, dass** sie in wasserfreier Form vorliegt.

26. Zusammensetzung nach einem der vorhergehenden Ansprüche, **dadurch gekennzeichnet, dass** sie als Lippenstift oder Lipgloss vorliegt.

27. Kosmetisches Verfahren, um einem Film einer kosmetischen Zusammensetzung die Eigenschaft der Nichtklebrigkeit, der Nichtmigration, des Glanzes und/oder des Komforts zu gelben, das darin besteht, in die Zusammensetzung i) mindestens einen Polyester, der durch Veresterung eines aliphatischen Hydroxycarbonsäureesters, der mindestens zwei Hydroxygruppen enthält, mit einer Polycarbonsäure gebildet wird, wobei die Polycarbonsäure ein Disäuredimer ist, das ausgehend von mindestens einer ungesättigten Fettsäure gebildet wird, und ii) mindestens einen Kohlenwasserstoffester, der von dem Polyester verschieden ist, einzubringen,

28. Verwendung der Kombination aus i) mindestens einem Polyester, der durch Veresterung eines aliphatischen Hydroxycarbonsäureesters, der mindestens zwei Hydroxygruppen enthält, mit einer Polycarbonsäure gebildet wird, wobei die Polycarbonsäure ein Disäuredimer ist, das ausgehend von mindestens einer ungesättigten Fettsäure gebildet wird, und ii) mindestens einem Kohlenwasserstoffester, der von dem Polyester verschieden ist, in einer physiologisch akzeptablen, nichtklebrigen, nichtmigrierenden, glänzenden, gut haltbaren und/oder angenehmen Zusammensetzung.

29. Verwendung der Kombination aus i) mindestens einem Polyester, der durch Veresterung eines aliphatischen Hydroxycarbonsäureesters, der mindestens zwei Hydroxygruppen enthält, mit einer Polycarbonsäure gebildet wird, wobei die Polycarbonsäure ein Disäuredimer ist, das ausgehend von mindestens einer ungesättigten Fettsäure gebildet wird, und ii) mindestens einem Kohlenwasserstoffester, der von dem Polyester verschieden ist, in einer physiologisch akzeptablen Zusammensetzung als Stoff, um der Zusammensetzung die Eigenschaften der Beständigkeit, der Nichtklebrigkeit, der Nichtmigration, des Glanzes und/oder des Komforts zu geben.
